## Europäisches Patentamt
## European Patent Office
## Office européen des brevets

(19)

(11) Veröffentlichungsnummer: **0 027 874**
**A1**

(12) **EUROPÄISCHE PATENTANMELDUNG**

(21) Anmeldenummer: **80105146.7**

(22) Anmeldetag: **29.08.80**

(51) Int. Cl.³: **C 07 C 99/12**, C 07 C 101/08, C 07 C 101/72, C 07 D 209/20, B 01 D 15/04

(30) Priorität: **30.10.79 DE 2943761**

(43) Veröffentlichungstag der Anmeldung: **06.05.81** Patentblatt 81/18

(84) Benannte Vertragsstaaten: **BE CH FR GB IT LI NL SE**

(71) Anmelder: **Degussa Aktiengesellschaft, Degussa AG Fachbereich Patente Rodenbacher Chaussee 4 Postfach 1345, D-6450 Hanau 1 (Stadtteil Wolfgang) (DE)**

(72) Erfinder: **Lüssling, Theodor, Dr.Dipl.-Chem., Zum Purren 16, D-7750 Konstanz (DE)**
Erfinder: **Scherberich, Paul, Dr.Dipl.-Chem., Köningsberger Strasse 8, D-7750 Konstanz (DE)**

(54) **Verfahren zur Isolierung von Aminosäuren, die mindestens einen aromatischen oder heteroaromatischen Ring enthalten.**

(57) Aminosäuren, die mindestens einen aromatischen oder heteroaromatischen Ring enthalten, werden aus ihren Lösungen isoliert, indem man sie an makroporöse, stark saure Ionenaustauscher mit einem mittleren Porendurchmesser von mindestens 10 nm adsorbiert. Die Verwendung solcher Ionenaustauscher ermöglicht es, die genannten Aminosäuren unter Verwendung von nur geringen Mengen an Elutionsmittel nahezu quantitativ zu eluieren.

EP 0 027 874 A1

0027874

DEUTSCHE GOLD- UND SILBER-SCHEIDEANSTALT VORMALS ROESSLER

Weissfrauenstrasse 9, 6000 Frankfurt 1

Verfahren zur Isolierung von Aminosäuren,
die mindestens einen aromatischen oder
heteroaromatischen Ring enthalten

Gegenstand der Erfindung ist ein Verfahren zur Isolierung von Aminosäuren, die mindestens einen aromatischen oder heteroaromatischen Ring enthalten, aus wässrigen, wässrig-alkoholischen oder alkoholischen Lösungen durch Adsorption an stark saure Ionenaustauscher und anschliessende Elution mit wässriger Ammoniaklösung.

Die Isolierung von Aminosäuren aus wässrigen Lösungen durch Adsorption an Ionenaustauscher und anschliessende Elution ist bekannt (Naturwissenschaften, 30, S. 87 (1942); Proc.Soc.Exptl.Biol.Med. 51, S. 252 (1942)).

Durch Einsatz der üblicherweise verwendeten gelartigen stark sauren Kationenaustauscherharze gelingt es, aliphatische Aminosäuren nahezu quantitativ zu isolieren. Dieses Verfahren eignet sich jedoch nicht zur Isolierung von aromatischen oder heteroaromatischen Aminosäuren, da diese infolge starker van der Waals'scher Adsorptionskräfte von dem Ionenaustauscherharz auch bei Einsatz eines grossen Überschusses an Elutionsmittel nur unvollständig eluiert werden können (Ann.N.Y.Acad. Sci. $\underline{47}$, S. 156 (1946); Biochem. J. $\underline{44}$, S. 521 (1949); Z.physiol.Ch. $\underline{282}$, S. 271 (1947)).

Das erfindungsgemässe Verfahren ist nun dadurch gekennzeichnet, dass man makroporöse Ionenaustauscher mit einem mittleren Porendurchmesser von mindestens 10 nm einsetzt.

Mit Hilfe des erfindungsgemässen Verfahrens gelingt es überraschenderweise, aromatische und heterocyclische Aminosäuren unter Verwendung von nur geringen Mengen an Elutionsmittel nahezu quantitativ zu isolieren. Es eignet sich zur Isolierung aromatischer und heteroaromatischer Aminosäuren sowie deren kernsubstituierter Derivate, wie z.B. Phenylglycin, 4-Hydroxy- oder 4-Methoxy-Phenylglycin; Phenylalanin, 4-Methyl-, 2,3-Dimethyl-, 4-Isopropyl-, 4-Chlor-, 4-Fluor-, 2-Brom-, 4-Nitro-, 4-Amino-, 4-Methoxy-, 4-Acetoxy-, 3,4-Methylendioxy-, 3,4-Dihydroxy-, 2-Hydroxy- oder 4-Mercapto-Phenylalanin; Tyrosin, Thyroxin, Thyronin, Phenylserin, Kynurenin, Tryptophan, 2-Methyl-, 5-Methyl-, 2-Hydroxy- oder 5-Hydroxy-Tryptophan; Furylalanin, Thienylalanin, Naphthylalanin oder Pyridylalanin.

Die genannten Aminosäuren können allein oder im Gemisch als solche oder in Form von Salzen, z.B. in Form der Hydrohalogenide, Sulfate oder Hydrogensulfate, oder als Salze mit beliebigen organischen Säuren in Lösung vorliegen. Es kann auch in Gegenwart von anorganischen Salzen wie NaCl, $NH_4Cl$, $Na_2SO_4$, $NaHSO_4$ oder Schwermetallsalzen, wie $FeCl_3$, $MnCl_2$ oder $CoCl_2$, gearbeitet werden. Gleichzeitig in Lösung anwesende organische Säuren stören nicht. Ebenso gelingt die Isolierung der genannten Aminosäuren bei Anwesenheit der entsprechenden N-acylierten Derivate, z.B. der N-Formyl-, N-Acetyl- oder N-Benzoyl-Derivate. Es können sowohl die Racemate als auch die optischen Isomeren eingesetzt werden. Die Konzentration der Aminosäuren oder von deren Gemischen ist nach oben nur durch ihre Löslichkeit begrenzt. Vorzugsweise erfolgt die Abtrennung bzw. Isolierung der Aminosäuren aus wässriger Lösung. Es kann jedoch auch in Anwesenheit von niederen aliphatischen Alkoholen, wie Methanol, Äthanol, n-Propanol oder Isopropylalkohol, gearbeitet werden.

Zur Isolierung von aromatischen oder heteroaromatischen Aminosäuren aus Lösungen nach dem erfindungsgemässen Verfahren eignen sich alle stark sauren Ionenaustauscher, die nach VGB Kraftwerkstechnik 55, (1), S. 40 (1975) als makroporös gelten, also einen mittleren Porendurchmesser von mindestens 10 nm besitzen. Die Verwendung von Ionenaustauschern mit einem mittleren Porendurchmesser von mehr als 200 nm bringt keine erkennbaren Vorteile mehr. Die Austauscher werden vorzugsweise in der $H^+$-Form eingesetzt. Bevorzugt werden makroporöse Kationenaustauscher auf der Grundlage von Styrol-Divinylbenzol-Copolymerisaten mit Sulfosäuregruppen eingesetzt. Zur Elution der adsorbierten Aminosäuren wird wie üblich eine wässrige Ammoniaklösung mit einer Konzentration von 3 bis 6 Gewichtsprozent verwendet.

Die bei der Adsorption und Elution einzuhaltenden Temperaturen und Drücke sind weitgehend beliebig. Normalerweise arbeitet man bei Temperaturen zwischen etwa 0° und etwa 100°C bei Normaldruck. Bei einer bevorzugten Arbeitsweise wird der in einer Säule befindliche makroporöse Ionenaustauscher von oben nach unten mit der Lösung der zu isolierenden Aminosäure bis zur vollständigen Beladung beschickt. Nach Waschen mit Wasser wird die adsorbierte Aminosäure mit einer ca. 6 gewichtsprozentigen wässrigen Ammoniaklösung eluiert und durch Einengen der Lösung isoliert. Nach Waschen des Austauschers wird dieser mit einer beispielsweise 6 gewichtsprozentigen wässrigen Salzsäure regeneriert.

Beispiel 1

Eingesetzt wurden 1000 ml eines makroporösen stark sauren Kationenaustauschers in der $H^+$-Form mit einer Totalkapazität von 1,8 val/l und einem mittleren Porendurchmesser von 65 nm (bestimmt nach der BET-Methode; J.Am.Chem.Soc. 60 (1938), S. 309 - 319). Der Austauscher befand sich in einer Glassäule von 5 cm Durchmesser, die Füllhöhe betrug 50 cm. Der Austauscher wurde von oben mit einer Lösung von 160 g L-Tryptophan in 16 l Wasser mit einem konstanten

0027874

Fluss von 12 l/h beschickt. Das Harz wurde anschliessend mit 1 l Wasser gewaschen. Das ablaufende Wasser war frei von L-Tryptophan. Zur Elution wurden 4 l 6 gewichtsprozentiger wässriger Ammoniaklösung verwendet. Durch Einengen wurden aus dem Eluat 156,8 g L-Tryptophan gewonnen. Dies entspricht einer Ausbeute von 98 % der Theorie.

Vergleichsversuch 1

Eingesetzt wurden 1000 ml eines stark sauren gelförmigen Kationenaustauschers in der $H^+$-Form mit einer Totalkapazität von 2,0 val/l und einer mittleren Porengrösse von 2 nm. Der Austauscher wurde wie im Beispiel 1 mit 160 g L-Tryptophan beladen und gewaschen. Zur Elution des adsorbierten L-Tryptophans wurde wieder eine 6 gewichtsprozentige wässrige Ammoniaklösung verwendet. Das Eluat wurde jeweils durch Eindampfen aufgearbeitet. Es wurden die folgenden Fraktionen erhalten:

| 6 %ige NH₃-Lösung (l) | L-Tryptophan (g) | [% d.Th.] |
|---|---|---|
| 10 | 100,0 | [62,5] |
| 10 | 18,4 | [11,5] |
| 10 | 5,2 | [3,2] |
| 10 | 2,5 | [1,5] |

Insgesamt wurden bei Einsatz von 40 l Elutionsmittel 126,1 g des adsorbierten L-Tryptophans eluiert. Dies entspricht einer Ausbeute von 78,7 % der Theorie.

Vergleichsversuch 2
_____

Es wurde wie beim Vergleichsversuch 1 verfahren, jedoch erfolgte die Elution mit insgesamt 40 l 6 gewichtsprozentiger wässriger Ammoniaklösung bei 40 bis 50$^{o}$C. Es wurden insgesamt 131,5 g des adsorbierten L-Tryptophans eluiert. Dies entspricht einer Ausbeute von 80 % der Theorie.

Beispiel 2
_____

Zum Vergleich wurden der in Beispiel 1 beschriebene makroporöse Kationenaustauscher (Austauscher I) und der im Vergleichsversuch 1 beschriebene gelförmige Kationenaustauscher (Austauscher II) eingesetzt. Die beiden Austauscher wurden jeweils mit einer Lösung von 130 g L-Phenylalanin in 4400 ml Wasser beschickt.

Es wurden folgende Elutionsmittelmengen und Ausbeuten ermittelt:

| Austauscher | NH$_3$-6 %ig (1) | Ausbeuten (g) | [% d.Th.] |
|---|---|---|---|
| Austauscher I | 3 | 130,0 | [100,0] |
| Austauscher II | 25 | 104,4 | [ 80,3] |

Beispiel 3
───────────

Eingesetzt wurden 1000 ml eines makroporösen stark sauren Kationenaustauschers mit einer Totalkapazität von 1,6 val/l und einem mittleren Porendurchmesser von 70 nm (Austauscher I) und 1000 ml eines gelförmigen stark sauren Kationenaustauschers mit einer Totalkapazität von 1,8 val/l und einem mittleren Porendurchmesser von 3 nm (Austauscher II). Die beiden Austauscher wurden jeweils mit einer Lösung von 30 g L-Tyrosin und 15 ml konzentriertem wässrigem Ammoniak in 20 l Wasser beschickt.

Es wurden folgende Elutionsmittelmengen und Ausbeuten ermittelt:

| Austauscher | NH$_3$-6 %ig (1) | Ausbeuten (g) | [% d.Th.] |
|---|---|---|---|
| Austauscher I | 1,5 | 29,1 | [97] |
| Austauscher II | 7,5 | 23,7 | [79] |

BAD ORIGINAL

Beispiel 4

Eingesetzt wurden die in Beispiel 3 beschriebenen stark sauren Kationenaustauscher I und II. Sie wurden beschickt mit Lösungen von jeweils 50 g DL-Phenylserin und 30 ml konzentrierter Ammoniak-Lösung in 1000 ml Wasser.

Es wurden folgende Elutionsmittelmengen und Ausbeuten ermittelt:

| Austauscher | $NH_3$-6 %ig (ml) | Ausbeuten (g) | [% d.Th.] |
|---|---|---|---|
| Austauscher I | 800 | 49,0 | [98] |
| Austauscher II | 5500 | 42,5 | [85] |

Beispiel 5

Eingesetzt wurden jeweils 2000 ml der in Beispiel 3 beschriebenen stark sauren Ionenaustauscher. Sie wurden beschickt mit Lösungen von jeweils 82,8 g L-Tryptophan, 107,6 g N-Acetyl-D-Tryptophan-Na-Salz und 32,8 g Natriumacetat in 5400 ml Wasser.

Folgende Elutionsmittelmengen und Ausbeuten an L-Tryptophan wurden ermittelt:

| Austauscher | $NH_3$-6 %ig (1) | Ausbeuten (g) | [% d.Th.] |
|---|---|---|---|
| Austauscher I | 1,7 | 81,7 | [98,7] |
| Austauscher II | 16,0 | 67,0 | [81] |

Beispiel 6
_____

Es wurden die in Beispiel 5 beschriebenen Austauscher eingesetzt. Sie wurden beschickt mit Lösungen von jeweils 69 g L-Phenylalanin, 113,3 N-Acetyl-D-Phenylalanin-Na-Salz und 34 g Natriumacetat in 2300 ml Wasser.

Folgende Elutionsmittelmengen und Ausbeuten an L-Phenylalanin wurden ermittelt:

| Austauscher | $NH_3$-6 %ig (1) | Ausbeuten (g) | $/$% d.Th.$/$ |
|---|---|---|---|
| Austauscher I | 1,5 | 66,7 | $/$97,8$/$ |
| Austauscher II | 12 | 51,7 | $/$75$/$ |

29. Oktober 1979
PAT/Dr.Sib-El

DEUTSCHE GOLD- UND SILBER-SCHEIDEANSTALT VORMALS ROESSLER
Weissfrauenstrasse 9, 6000 Frankfurt 1

Patentanspruch:

Verfahren zur Isolierung von Aminosäuren, die mindestens einen aromatischen oder heteroaromatischen Ring enthalten, aus wässrigen, wässrig-alkoholischen oder alkoholischen Lösungen durch Adsorption an stark saure Ionenaustauscher und anschliessende Elution mit wässriger Ammoniaklösung, dadurch gekennzeichnet, dass man makroporöse Ionenaustauscher mit einem mittleren Porendurchmesser von mindestens 10 nm einsetzt.

Europäisches
Patentamt

**EUROPÄISCHER RECHERCHENBERICHT**

Nummer der Anmeldung

002787 4

EP 80105146.7

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 1 493 900 (KYOWA HAKKO KOGYO)<br>* Seiten 12,13 *<br>-- | 1 |
| A | DE - A - 2 333 375 (OY MEDICA)<br>* Ansprüche *<br>-- | 1 |
| | DE - A - 2 241 414 (OY MEDICA)<br>* Beispiel *<br>-- | 1 |
| | DE - A - 1 595 868 (KYOWA HAKKO KOGYO)<br>* Beispiele *<br>---- | 1 |

**KLASSIFIKATION DER ANMELDUNG (Int Cl )**

C 07 C 99/12

C 07 C 101/08

C 07 C 101/72

C 07 D 209/20

B 01 D 15/04

**RECHERCHIERTE SACHGEBIETE (Int. Cl.)**

C 07 C 99/00

C 07 C 101/00

C 07 D 209/00

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 10-12-1980 | HOFBAUER |

EPA form 1503.1  06.78